# EUROPÄISCHE PATENTSCHRIFT

(11) **EP 1 996 318 B1**
(45) Veröffentlichungstag und Bekanntmachung des Hinweises auf die Patenterteilung: **07.09.2011**
(21) Anmeldenummer: 06829213.5
(22) Anmeldetag: 30.11.2006
(51) Int. Cl.: B01J 4/00, B01J 19/24, B01F 3/04

(54) **SCHLAUFENREAKTOR MIT VERSTOPFUNGSRESISTENTER GASVERTEILUNG**
LOOP REACTOR WITH BLOCKAGE-RESISTANT GAS DISTRIBUTION
REACTEUR EN BOUCLE DOTE D'UNE REPARTITION DE GAZ ANTI-COLMATAGE

(30) Priorität: 05.12.2005 DE 102005057977
(43) Veröffentlichungstag der Anmeldung: 03.12.2008
(73) Patentinhaber: STRABAG Umweltanlagen GmbH, 01069 Dresden (DE)
(72) Erfinder: MEYER, Thorsten, 01665 Klipphausen (DE); LANGHANS, Gerhard, 01159 Dresden (DE)
(86) Internationale Anmeldenummer: PCT/EP2006/011516
(87) Internationale Veröffentlichungsnummer: WO 2007/065596

(56) Entgegenhaltungen:
- EP-A2- 0 057 659
- US-A- 3 400 051
- US-A- 3 813 086
- US-A- 5 650 070

## Beschreibung

Die Erfindung betrifft einen Schlaufenreaktor gemäß dem Oberbegriff des Anspruch 1.

Schlaufenreaktoren werden in der Verfahrenstechnik vielfach eingesetzt. Dabei werden flüssige oder fließfähige Medien in einem Reaktor unter Umwälzung des Reaktorinhalts behandelt. Der Reaktorinhalt wird durch eine zentrale Leiteinrichtung, z.B. ein senkrechtes Leitrohr, im Reaktorinnenraum z.B. von unten nach oben geleitet und strömt außerhalb des Leitrohrs wieder nach unten. Zur Induktion der Aufwärtsströmung im Leitrohr wird ein Gas im unteren Bereich des Leitrohrs eingeleitet. Der Reaktorinhalt wird somit in Form einer Schlaufenströmung im Reaktor umgewälzt.

Derartige Schlaufenreaktoren mit innenliegender Schlaufe werden als volldurchmischte anaerobe Suspensionsreaktoren insbesondere zur Biogasgewinnung aus biologisch abbaubaren Substanzen enthaltenden Suspensionen, z.B. Bioabfall oder nachwachsende Rohstoffe, eingesetzt. Dieses Prinzip ist z.B. unter anderem in der CH 31 67 67 oder der DE197 25 823 A1 beschrieben. Dabei wird abgetaucht in das zentrale Leitrohr Biogas eingeblasen. Durch die abgesenkte Gemischdichte im Leitrohr relativ zum äußeren Flüssigkeitsstand entsteht ein Druckunterschied in der Flüssigkeit, der eine Aufströmung im Leitrohr induziert. Das eingedüste Biogas soll eine möglichst gleichmäßige und feinblasige Verteilung über den Leitrohrquerschnitt erfahren, um eine effiziente Gemischbildung zu gewährleisten.

Bei Leitrohren geringen Durchmessers wird häufig außen eine gasführende Ringleitung aufgesetzt, aus der über Bohrungen Gas innen am Leitrohrumfang austritt. Für Leitrohre größeren Durchmessers ist dieses Prinzip ungünstig, da keine Verteilung über den Querschnitt erreicht wird. Zentrische Eindüsungen am Boden des Reaktors unter dem Leitrohreintritt sind für große Gärreaktoren ebenfalls ungünstig, da sie bei Gaseindüsungen tiefer als ca. 14m unter der Flüssigkeitsoberfläche aufgrund des statischen Drucks keinen signifikanten Beitrag mehr für die treibende Druckdifferenz bringen. Die Verdichterleistung zum Eintrag des Gases in der entsprechenden Tiefe muss jedoch aufgebracht werden.

Aus diesen Gründen werden bei großen Reaktoren häufig vom Reaktordach abgetauchte Lanzen eingesetzt, die das Biogas bevorzugt zwischen 7m und 12m unter dem Flüssigkeitsspiegel eintragen. Dabei gibt es nach dem Stand der Technik zwei Alternativen mit den nachstehend beschriebenen Vor- und Nachteilen:

Die eine Alternative sieht den Einsatz eines zentralen Gaszuführrohres mit am unteren Ende angesetzten 3 bis 7, bevorzugt 5, Gasverteilerrohren kleineren Durchmessers vor. Die Gasverteilerrohre gewährleisten den Gasaustritt auf dem gewünschten Teilkreis innerhalb des Leitrohres und sollen so für eine effiziente Gemischbildung sorgen. Dieses System hat den Vorteil, dass ein großkalibriges Zuführrohr für das Gas verwendet werden kann, das über die große Abtauchlänge biege- und schwingungssteif ausgeführt werden kann. Nachteilig sind in der Praxis jedoch die seitlich abgehenden Verteilerrohre kleinen Durchmessers, die sich gerade bei hochkonzentrierten Suspensionen, z.B. Faulschlämmen, mittelfristig mit Inkrustierungen und Anbackungen zusetzen können. Der dadurch asymmetrisch erfolgende Gasaustritt aus den noch freien Rohren führt zu empfindlichen Störungen des hydraulischen Systems.

Bei der anderen Alternative werden separat auf einem Teilkreis nach unten geführte Einzellanzen eingesetzt. Diese bieten eine bessere Reinigungsmöglichkeit, da sie vom Dach des Reaktors aus über die gesamte Länge bis zum Gasaustritt gereinigt werden können. Ihr Problem ist allerdings die ungenügende mechanische Stabilität aufgrund der kleineren Einzelrohrdurchmesser gegenüber dynamischen Beanspruchungen und von Schwimmdeckenteilen aufgeprägten Scherkräften im Betrieb.

Aus der US 5 650 070 A ist es bekannt, in einem aeroben Abwasserreaktor sauerstoffhaltiges Gas, insbesondere Luft, über ein Rohr einzublasen, wobei dieses Rohr an seinem Ende eine unten offene kegelförmige Aufweitung besitzt, über deren Rand hinweg das Gas dann in das Abwasser gelangt, um dort den Sauerstoff an das Abwasser abzugeben.

Der vorliegenden Erfindung liegt die Aufgabe zugrunde, die Gaszuführungs- und Gasverteileinrichtungen bei Schlaufenreaktoren so auszugestalten, dass einerseits Knick- und Biegesteifigkeit und andererseits eine effiziente Gasverteilung bei geringer Neigung zu Verstopfungen gewährleistet ist.

Diese Aufgabe wird erfindungsgemäß durch die Merkmale des Anspruch 1 gelöst.

Bisherige Konstruktionen gehen davon aus, Gas über Rohre bis zur Austrittsstelle zu leiten. Erfindungsgemäß wird dagegen eine Konstruktion vorgeschlagen, die mindestens eine zentrale Gaszufuhreinrichtung aufweist, welche beispielsweise aus einem stabilen zentralen Mittelrohr bestehen kann, das bis zur gewünschten Eintragstiefe im Schlaufenreaktor unter den Flüssigkeitsspiegel reicht. Die Gasverteilung von dieser zentralen Gaszufuhreinrichtung auf den gewünschten Austrittsteilkreis in der Leiteinrichtung erfolgt im Gegensatz zum Stand der Technik nicht über Verteilerrohre, sondern über unten offene Gasleitelemente, welche nicht verstopfen können. Vorzugsweise sind die Gasleitelemente als an der Unterseite offene halbkreisförmig gebogene Bleche ausgebildet. Die zentrale Gaszufuhreinrichtung ist zweckmäßigerweise als senkrechtes Zuführrohr ausgebildet, welches bis unter den im Schlaufenreaktor vorgesehenen Flüssigkeitsspiegel reicht. Vorzugsweise sind die Gasleitelemente außen an diesem Zuführrohr angebracht. Beispielsweise können sie bei Ausbildung des Zuführrohrs aus Metall und der Gasleitelemente als halbkreisförmig gebogene Bleche an dem Zuführrohr angeschweißt sein. Je nach Einsatzfall sind drei bis sieben, in der Regel bevorzugt fünf, Gasleitelemente in Höhe der Gasaustrittsöffnungen über den Rohrumfang verteilt am Zuführrohr angebracht. Die Gasaustrittsöffnungen im Zuführrohr ermöglichen so den Gasdurchtritt unter die Gasleitelemente. Diese sind bevorzugt in einem Winkel zwischen 5 Grad und 45 Grad gegen die Horizontale nach oben geneigt, wobei ein Neigungswinkel zwischen 10 Grad und 20 Grad besonders vorteilhaft ist. Durch die Auftriebskräfte strömt das Gas unter dem bevorzugt konvex gewölbten Gasleitelement entlang bis zur Außenkante, wo es als Blasenkette abreißt. Die Gasleitelemente weisen zweckmäßigerweise seitliche Führungsflächen auf, die im Bereich um die Gasauftrittsöffnungen der zentralen Gaszufuhreinrichtung verlängert nach unten gezogen sind. Dadurch wird vermieden, dass im Bereich des turbulenten Gasdurchtritts das Gas seitlich unter den Gasleitelementen austritt. Das Gas folgt somit sicher der Führungskontur der Gasleitelemente.

Nach Ingangkommen der großräumigen Reaktorzirkulationsströmung unterstützt die Flüssigkeitsaufstromgeschwindigkeit in der Leiteinrichtung, die prozessspezifisch zwischen 0,5m pro Sekunde und ca. 1,4m pro Sekunde beträgt, den Gasstrom unter den Gasleitelementen und stabilisiert die Ausbildung eines gewünschten Blasenspektrums kleiner Durchmesser zwischen 10mm und ca. 30mm durch die Scherkräfte an den Abreißkanten der Kontur. Gleichzeitig bewirkt die Flüssigkeits-Gas-strömung entlang der Gasleitelemente einen Selbstreinigungseffekt, in dem sie Ansätze von Anbackungen und Inkrustierungen abwäscht.

Die wesentlichen Vorteile der erfindungsgemäßen Vorrichtung bestehen insbesondere in der Robustheit und Verstopfungsresistenz des Gaseintragssystems, was insbesondere bei der Behandlung von feststoffreichen Suspensionen erst einen wirtschaftlichen Dauerbetrieb ermöglicht. Der Einsatz einer zentralen Gaszufuhreinrichtung, die als stabiles Zentralrohr ausgebildet sein kann, in Verbindung mit daran angebrachten nach unten offenen Gasleitelementen ergibt insgesamt ein Gaseintragssystem mit hoher Knick- und Biegesteifigkeit. Die zentrale Gaszufuhreinrichtung kann auf einfache Weise, z.B. durch Einführen einer Rohrbürste oder eines Hochdruckreinigungskopfes, gereinigt werden. Die nach unten offenen Gasleitelemente können nicht verstopfen, so dass insgesamt der Wartungsaufwand für das Gaseintragssystem minimiert werden kann.

Die Erfindung eignet sich für alle denkbaren Schlaufenreaktoren, bei denen der Reaktorinhalt über eine Leiteinrichtung umgewälzt wird, die mit einem Gas zur Ausbildung eines Treibstrahls beaufschlagt wird. Mit besonderem Vorteil kann die Erfindung bei Biogasreaktoren zur Behandlung von biologisch abbaubare Substanzen enthaltenden Suspensionen angewandt werden, bei denen z.B. Bioabfälle oder nachwachsende Rohstoffe vergoren werden. Insbesondere bei der Behandlung von nachwachsenden Rohstoffen in Biogasreaktoren besteht aufgrund des hohen Feststoffgehalts der zu behandelnden Suspension ein erhöhtes Verstopfungsrisiko für das Gaseintragssystem. Hier bietet die Erfindung entscheidende Vorteile.

Im Folgenden soll die Erfindung eines in den Figuren schematisch dargestellten Ausführungsbeispiels näher erläutert werden:

Es zeigen
- Figur 1: einen Querschnitt eines Gaseintragssystems für einen Schlaufenreaktor.
- Figur 2: eine Detailansicht eines Gasleitelements

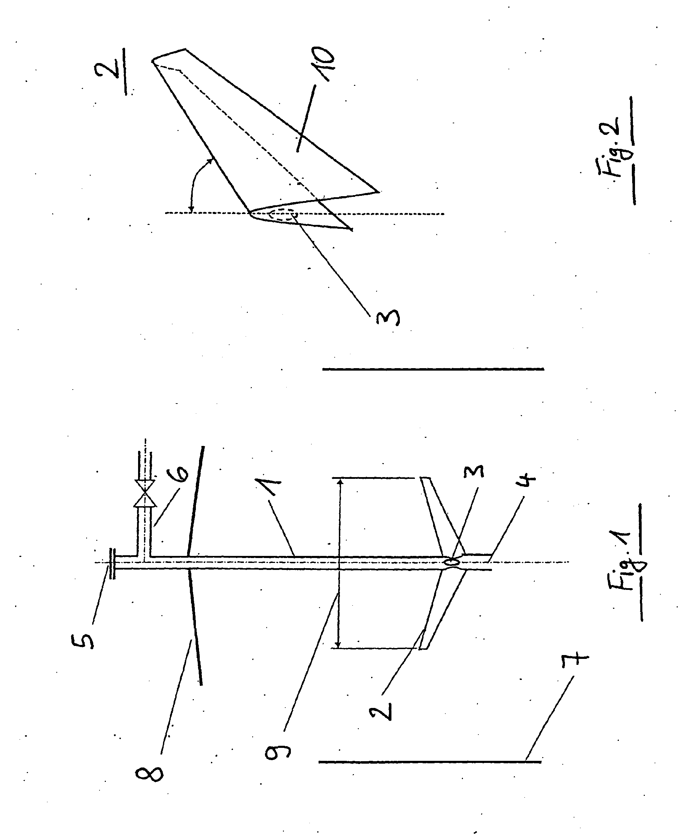

In der Figur 1 ist ein Ausschnitt aus einem Schlaufenreaktor gezeigt, der beispielsweise als volldurchmischter anaerober Suspensionsreaktor zur Behandlung von Bioabfällen oder nachwachsenden Rohstoffen eingesetzt werden kann. In dem Ausschnitt aus dem Schlaufenreaktor ist die als Leitrohr ausgebildete Leiteinrichtung 7 zur Ausbildung der innenliegenden Schlaufenströmung im Schlaufenreaktor dargestellt. Im Leitrohr 7 ist ein zentrales Zuführrohr 1 zum Einbringen des Gases in das Leitrohr 7 angeordnet. Das Zuführrohr 1 ist von oben durch das Reaktordach 8 als Lanze eingeführt. Unterhalb des vorgesehenen Flüssigkeitsspiegels im Schlaufenreaktor weist das Zuführrohr 1 als Bohrungen ausgeführte Gasaustrittsöffnungen 3 auf. In diesem Bereich sind Gasleitelemente 2, die als an der Unterseite offene halbkreisförmig gebogene Bleche ausgebildet sind, am Zuführrohr 1 angeschweißt. Über diese Gasverteilelemente 2 erfolgt die Gasverteilung vom zentralen Zuführrohr 1 auf den gewünschten Austrittsteilkreis 9 im Leitrohr 7. Dabei ermöglichen die Gasaustrittsöffnungen 3 im zentralen Zuführrohr 1 den Gasdurchtritt unter die Gasleitelemente 2, die in einem Winkel von 10 bis 20 Grad nach oben gerichtet sind. Durch die Auftriebskräfte strömt das Gas unter dem konvex gewölbten Gasleitelement bis zur Außenkante wo es als Blasenkette abreißt. Dabei bewirkt die Flüssigkeits- Gasströmung entlang der Gasleitelemente 2 einen Selbstreinigungseffekt, in dem sie Ansätze von Anbackungen und Inkrustierungen abwäscht. Das zentrale Zuführrohr 1 und der Bereich der Gasaustrittsöffnungen 3 unter den Gasleitelementen 2 lassen sich im Betrieb leicht reinigen. Dazu ist das unten offene zentrale Zuführrohr 1 100mm bis 300mm unter die Gasaustrittsöffnungen 3 als Führungsstrecke 4 verlängert. Über dem Reaktordach 8 endet das Zuführrohr 1 in bekannter Weise mit einem Blindflansch 5, unter dem seitlich die Gaszuführung 6 in das Zuführrohr 1 eingebunden ist. Im Reinigungsfall lässt sich diese Leitung über ein Ventil 9 vom Gassystem absperren. Dann kann über den entfernten Blindflanscheingang 5 ein Reinigungselement, beispielsweise in Form einer Rohrbürste oder eines Hochdruckreinigungskopfes, eingeführt und bis in den Bereich der Gasaustrittsöffnungen 3 abgesenkt werden. Auf diese Weise lassen sich Verblockungen sicher mechanisch/hydraulisch entfernen. Während der Reinigung erfolgt kein signifikanter Gasaustritt aus dem zentralen Zuführrohr 1, da es im unteren Bereich durch den Flüssigkeitsstand des Schlaufenreaktors sicher verschlossen ist. Die unten offene Führungsstrecke 4 am zentralen Zuführrohr 1 verhindert ein Hängenbleiben des Reinigungselementes bei der Arbeit in Höhe der Gasaustrittsöffnungen 3. Abgestoßene Feststoffe sammeln sich nicht im Zuführrohr 1, sondern sinken durch die untere Öffnung in die Reaktorflüssigkeit ab. Ein Gasaustritt erfolgt hier im Betriebszustand nicht, da die statische Flüssigkeitssäule das eingepresste Gas zwingt, aus den höher gelegenen Gasaustrittsöffnungen 3 unter die Gasleitelemente 2 zu strömen.

Figur 2 betrifft eine Detailansicht eines Gasleitelements. Es handelt sich dabei um ein konvex gebogenes Blech, das nach unten offen ist. Im Bereich der Gasaustrittsöffnung 3 sind die seitlichen Führungsflächen 10 des Gasleitelements verlängert nach unten gezogen. Dadurch wird vermieden, dass im Bereich des turbulenten Gasdurchtritts durch die Gasaustrittsöffnung 3 das Gas seitlich unter dem Gasleitelement 2 austritt. Das Gas folgt somit sicher der Führungskontur des Gaselements 2.

## Patentansprüche

1. Anaerober Schlaufenreaktor zur Herstellung von Biogas aus biologisch abbaubare Substanzen enthaltenden Suspensionen, insbesondere Bioabfällen oder nachwachsenden Rohstoffen, mit einer im Innenraum des Schlaufenreaktors angeordneten Leiteinrichtung (7) zur Umwälzung des Reaktorinhalts, wobei die Leiteinrichtung (7) mindestens eine zentrale Gaszufuhreinrichtung (1) mit daran angebrachten Gasverteileinrichtungen (2) zum Gaseintrag in die Leiteinrichtung (7) aufweist,
**dadurch gekennzeichnet, dass** die Gasverteileinrichtungen (2) als nach unten offene, als halbkreisförmig gebogene Bleche ausgeführte, Gasleitelemente ausgebildet sind, die oberhalb von Gasaustrittsöffnungen (3) der zentralen Gaszufuhreinrichtung (1) angeordnet sind,
und dass drei bis sieben solche Gasleitelemente (2) in Höhe der Gasaustrittsöffnungen (3) über den Rohrumfang verteilt am Zuführrohr (1) angebracht sind,
und dass die Gasleitelemente (2) in einem Winkel zwischen 5 Grad und 45 Grad gegen die Horizontale nach oben geneigt sind.

2. Schlaufenreaktor nach Anspruch 1, **dadurch gekennzeichnet, dass** die zentrale Gaszufuhreinrichtung (1) als senkrechtes Zuführrohr ausgebildet ist, welches bis unter den im Schlaufenreaktor vorgesehenen Flüssigkeitsspiegel reicht.

3. Schlaufenreaktor nach Anspruch 2, **dadurch gekennzeichnet, dass** die Gasleitelemente (2) außen am Zuführrohr (1) angebracht sind.

4. Schlaufenreaktor nach einem der Ansprüche 1 bis 3, **dadurch gekennzeichnet, dass** die Gasleitelemente (2) seitliche Führungsflächen (10) aufweisen, die im Bereich um die Gasaustrittsöffnungen (3) der zentralen Gaszufuhreinrichtung (1) verlängert nach unten gezogen sind.

## Claims

1. An anaerobic loop reactor for producing biogas from suspensions containing biologically degradable substances, in particular biological wastes or renewable raw materials, with a guide device (7) arranged in the interior of the loop reactor for circulating the reactor contents, wherein the guide device (7) has at least one central gas feed device (1) with gas distributor devices (2) mounted thereon for gas introduction into the guide device (7),
**characterized in that** the gas distributor devices (2) are designed as gas guiding elements open at the bottom, constructed as semicircularly bent plates, which are arranged above gas outlet orifices (3) of the central gas feed device (1),
and **in that** three to seven of these gas guiding elements (2) are mounted on the feed pipe (1) distributed around the pipe circumference at the level of the gas outlet orifices (3),
and **in that** the gas guiding elements (2) are upwardly inclined at an angle between 5 degrees and 45 degrees against the horizontal.

2. A loop reactor according to claim 1, **characterized in that** the central gas feed device (1) is designed as a vertical feed pipe which reaches down to below the liquid level intended in the loop reactor.

3. A loop reactor according to claim 2, **characterized in that** the gas guiding elements (2) are mounted externally on the feed pipe (1).

4. A loop reactor according to one of claims 1 to 3, **characterized in that** the gas guiding elements (2) comprise lateral guide surfaces (10) which are extended downwards in the area around the gas outlet orifices (3) of the central gas feed device (1).

## Revendications

1. Réacteur en boucle anaérobie pour la fabrication de biogaz à partir de suspensions contenant des substances biodégradables, en particulier des déchets biologiques ou des matières premières renouvelables, avec un dispositif de guidage (7) placé dans l'espace intérieur du réacteur en boucle pour le brassage du contenu du réacteur, en notant que le dispositif de guidage (7) présente au moins un dispositif d'amenée central du gaz (1) avec des dispositifs de répartition du gaz qui y sont raccordés (2) pour introduire le gaz dans le dispositif de guidage (7),
**caractérisé en ce que** les dispositifs de répartition de gaz (2) sont constitués comme des éléments de guidage de gaz ouverts vers le bas, réalisés sous forme de tôles pliées en demi-cercle, qui au-dessus des ouvertures de sortie de gaz (3) du dispositif central d'amenée de gaz (1),
et **en ce que** trois à sept de ces éléments de guidage de gaz (2) sont placés à hauteur des ouvertures de sortie de gaz (3) répartis sur le périmètre tubulaire du tuyau d'amenée (1),
et **en ce que** les éléments de guidage (2) sont inclinés vers le haut dans un angle compris entre 5 degrés et 45 degrés par rapport à l'horizontale.

2. Réacteur en boucle anaérobie selon revendication 1, **caractérisé en ce que** le dispositif central d'amenée de gaz (1) est constitué comme un tube d'amenée vertical, qui parvient jusqu'en dessous du niveau de liquide prévu dans le réacteur en boucle.

3. Réacteur en boucle anaérobie selon revendication 2, **caractérisé en ce que** les éléments de guidage de gaz (2) sont situés à l'extérieur sur le tube d'amenée (1).

4. Réacteur en boucle anaérobie selon les revendications 1 à 3 **caractérisé en ce que** les éléments de guidage (2) présentent des surfaces de guidage latérales (10), qui dans la zone autour des ouvertures de sortie d'air (3) du dispositif central d'amenée de gaz (1) sont tirés et prolongés vers le bas.
